Europäisches Patentamt

European Patent Office

Office européen des brevets

(19)

(11) Veröffentlichungsnummer: **0 206 128 B1**

## (12) EUROPÄISCHE PATENTSCHRIFT

(45) Veröffentlichungstag der Patentschrift: **13.11.91**

(21) Anmeldenummer: **86107988.7**

(22) Anmeldetag: **11.06.86**

(51) Int. Cl.⁵: **B01F 17/52**, A61K 7/00, A61K 9/10, A61K 47/00

Die Akte enthält technische Angaben, die nach dem Eingang der Anmeldung eingereicht wurden und die nicht in dieser Patentschrift enthalten sind.

(54) **Kosmetische Zubereitungen mit verbessertem Viskositätsverhalten.**

(30) Priorität: **18.06.85 DE 3521713**

(43) Veröffentlichungstag der Anmeldung:
**30.12.86 Patentblatt 86/52**

(45) Bekanntmachung des Hinweises auf die Patenterteilung:
**13.11.91 Patentblatt 91/46**

(84) Benannte Vertragsstaaten:
**AT BE CH DE FR GB IT LI NL SE**

(56) Entgegenhaltungen:
**US-A- 3 485 915**

**Ullmanns Encyklopädie der technischen Chemie,4. Aufl., Band 10, S. 449**

(73) Patentinhaber: **Henkel Kommanditgesellschaft auf Aktien
Postfach 1100 Henkelstrasse 67
W-4000 Düsseldorf-Holthausen(DE)**

(72) Erfinder: **Ansmann, Achim, Dr.
Fichtestrasse 19
W-4010 Hilden(DE)**

(74) Vertreter: **von Kreisler, Alek, Dipl.-Chem. et al
Patentanwälte Von Kreisler-Selting-Werner,
Deichmannhaus am Hauptbahnhof
W-5000 Köln 1(DE)**

EP 0 206 128 B1

**Beschreibung**

Gegenstand der Erfindung sind kosmetische Zubereitungen für die topische Anwendung in Form einer Öl in Wasser Emulsion (im folgenden O/W-Emulsion).

Die Viskosität und das rheologische Verhalten von O/W-Emulsionen werden einerseits durch die Art, Menge und Feinteiligkeit der dispersen Phase, andererseits auch durch die Zusammensetzung der kontinuierlichen wäßrigen Phase bestimmt. Bei Emulsionen, die für haut- und haarkosmetische topische Anwendungen bestimmt sind, hat das rheologische Verhalten einen erheblichen Einfluß auf das subjektive Empfinden bei der Anwendung.

Insbesondere bei Emulsionen für die kosmetische Anwendung auf der Haut erwartet der Verbraucher einen taktil gehaltvollen Eindruck beim Verreiben auf der Haut; eine Erhöhung des Anteils an Ölphase führt aber oft zu einem unerwünschten fettigen Hautgefühl.

Man hat daher zu Steuerung der Viskosität der wäßrigen Phase wasserlösliche Polymere, sogenannte Hydrokolloide zugegeben. Bekannt ist bei kosmetischen und pharmazeutischen topischen O/W-Emulsionen die Verwendung von wasserlöslichen Pflanzengummen wie Alginaten und Traganth, von wasserlöslichen Cellulosederivaten wie Carboxymethylcellulose, Hydroxyethylcellulose, Methylcellulose, von wasserlöslichen Stärkederivaten und von synthetischen Polymeren wie z. B. von Polyvinylalkohol, Polyethylenoxiden oder Polyacrylsäuren zur Verdickung der wäßrigen Phase und zur Stabilisierung der Emulsionen.

Wäßrige Lösungen dieser Polymeren sind, wie die Lösungen der meisten wasserlöslichen Polymeren, strukturviskos, d. h. daß die Viskosität unter dem Einfluß von Scherkräften abnimmt. O/W-Emulsionen, deren wäßrige Phase durch solche strukturviskosen Polymeren verdickt ist, haben den Nachteil, daß bei dem Vorgang des Verreibens der Emulsion auf der Haut, infolge der beim Verreiben auftretenden Scherkräfte von der Viskosität wenig zu spüren ist, so daß die Emulsionen gehaltlos und kosmetisch wenig ansprechend wirken.

US-A-3 485 915 betrifft wäßrige und/oder alkoholische Zusammensetzungen, die zur topischen Applikation auf die Haut geeignet sind und die als Verdicker ein neutralisiertes Acrylsäurepolymer und Hydroxypropylcellulose enthalten.

Es bestand daher die Aufgabe, weniger scherabhängige Verdikkungsmittel für die wäßrige Phase von O/W-Emulsionen aufzufinden und O/W-Emulsionen zu schaffen, die ohne zusätzliche Erhöhung der Ölphase mehr "Körper", also ein verbessertes Viskositätsverhalten und ein kosmetisch befriedigenderes Verhalten beim Auftragen und Verteilen auf der Haut oder auf dem Haar aufweisen.

Gegenstand der Erfindung ist eine kosmetische Zubereitung für die topische Anwendung in Form einer Öl-in-Wasser-Emulsion, die einen wasserlöslichen nichtionischen Celluloseether und ein vernetztes Acrylsäure-Polymerisat oder -Copolymerisat enthält, dadurch gekennzeichnet, daß die kontinuierliche wäßrige Phase eine Kombination von Verdickungsmitteln aus

(a) Methylhydroxypropylcellulose mit einer Brookfield-Viskosität von 40 bis 40 000 mPas, gemessen bei 20 UpM, einer 2 Gew.-%igen wäßrigen Lösung des Celluloseethers bei 20 °C und

(b) einem wasserlöslichen Salz eines vernetzten Acrylsäure-Polymerisats oder Acrylsäure-Copolymerisats mit einem mittleren Molekulargewicht von 1 000 000 bis 5 000 000 im Mengenverhältnis (a) : (b) = 9 : 1 bis 1 : 9

enthält.

Die Erfindung beruht auf der Beobachtung, daß wäßrige Lösungen der Kombination dieser Hydrocolloide eine erhöhte Viskosität insbesondere unter dem Einfluß von Scherkräften aufweisen, die gegenüber der Viskosität der Lösung der einzelnen Komponenten synergistisch gesteigert ist.

Als wasserlöslicher nichtionischer Celluloseether wird erfindungsgemäß Methylhydroxypropylcellulose eingesetzt. Methylhydroxypropylcellulosen sind im Handel z. B. unter dem Warenzeichen VISCONTRAN [R]MHPC erhältlich.

Die verschiedenen handelsüblichen Typen solcher nichtionogener Celluloseether unterscheiden sich durch den Substitutionsgrad und den Abbaugrad der Cellulose (d. h. das mittlere Molekulargewicht), wobei sich Qualitäten mit unterschiedlicher Lösungsviskosität ergeben.

Die erfindungsgemäß eingesetzten Methylhydroxypropylcellulosen haben in 2-gew.-%iger wäßriger Lösung bei 20 °C eine Viskosität von 40 bis 40 000 mPas (gemessen mit einem Brookfield-Rotationsviskosimeter bei 20 UpM).

Als vernetzte Acrylsäure-Polymerisate eignen sich Produkte, die durch Copolymerisation von Acrylsäure mit 0,1 - 4,0 Gew.-% eines Polyalkenyl-polyethers eines mehrwertigen Alkohols mit mehr als einer Alkenylethergruppe im Molekül als Vernetzungsmittel erhalten werden. Ein Beispiel für ein solches Vernetzungsmittel ist z. B. Polyallylsucrose.

Gegebenenfalls können bei der Herstellung der vernetzten Acrylsäure-Polymerisate auch weitere Comonomere in Mengen bis zu 59 Gew.-% der Monomerenmischung eingesetzt werden. Geeignete Comonomere sind z. B. Maleinsäureanhydrid, N-Methylacrylamid, Methyl-vinylether oder Mischungen solcher zusätzlicher Monomerer. Solche vernetzte Acrylsäurepolymerisate sind z. B. aus US-A-2.798.053 bekannt und unter dem Warenzeichen CARBOPOL (R) im Handel. Die vernetzten Acrylsäure-Polymerisatelassen sich in Wasser dispergieren, die starke Verdickungswirkung wird jedoch erst erreicht, wenn die Polymerisate durch anorganische Basen wie z. B. Natriumhydroxid, Kaliumhydroxid, Ammoniak oder durch niedermolekulare Amine oder Alkanolamine in die Salzform überführt werden.

Geeignete Acrylsäure-Polymerisate haben in 1gew.-%iger wäßriger Lösung, neutralisiert mit Natronlauge bei einem pH-Wert von 7 - 8 bei 20 °C eine Viskosität von 1 000 - 100 000 mPas (gemessen mit einem Brookfield-Rotationsviskosimeter bei 20 UpM).

Erfindungsgemäße Kosmetische Zubereitungen enthalten Öl-in-Wasser-Emulsionen mit einer diskontinuierlichen, (inneren) Ölphase, die im wesentlichen aus kosmetischen Ölkomponenten, Fetten und/oder Wachsen, öllöslichen Emulgatoren und gegebenenfalls öllöslichen kosmetischen Wirkstoffen besteht.

Als kosmetische Ölkomponenten eignen sich alle hierfür bekannten pflanzlichen, tierischen, mineralischen und synthetischen Öle. Als Beispiele seien genannt: Olivenöl, Sonnenblumenöl, Maiskeimöl, Spermöl, Nerzöl, Paraffinöl, Silikonöle, (z. B. Dimethylpolysiloxan) Squalan, Oleylalkohol, 2-Octyl-dodecanol, Decyloleat, Isopropylmyristat, Isononyl-stearat, 2-Ethylhexyl-palmitat, Glycerin-tricaprylat und andere als kosmetische Ölkomponenten bekannte Ester, Alkohole oder Kohlenwasserstoffe. Als kosmetische Fette und Wachse eignen sich alle hierfür bekannten Produkte mit Schmelzpunkten bis ca. 80 °C, als Beispiele seien genannt gehärtete pflanzliche und tierische Fette (Triglyceride), Walrat, Fettalkohole, z. B. Cetylalkohol, Stearylalkohol, Ester wie z. B. Cetylpalmitat und natürliche Wachse wie z. B. Wollwachs, Bienenwachs, Japanwachs, Carnaubawachs, Candelillawachs, mineralische Wachse wie z. B. Montanwachs, Paraffine, Vaseline sowie synthetische Paraffine wie z. B. die Polyethylenwachse.

Als öllösliche Emulgatoren eignen sich alle für die Emulgierung der vorgenannten Öle, Fette und Wachse geeigneten, öllöslichen Emulgatoren. Als Beispiele seien genannt die Seifen von Fettsäuren mit 12 - 22 C-Atomen, die Mono- und Diglyceride und die Sorbitan-Partialester von Fettsäuren mit 12 - 22 C-Atomen und die Anlagerungsprodukte von 2 - 30 Mol Ethylenoxid an solche Fettsäure-Partialglyceride und Sorbitanfettsäureester, die Anlagerungsprodukte von 2 - 30 Mol Ethylenoxid an Fettalkohole mit 12 - 22 C-Atomen, an Fettsäuren mit 12 - 22 C-Atomen, an Alkylphenole mit 8 - 16 C-Atomen in der Alkylgruppe und an Fettsäurealkanolamide, die Fettalkoholsulfate mit 16 - 22 C-Atomen in Form ihrer Alkali- oder Alkanolammoniumsalze, die Phosphorsäureester linearer Fettalkohole oder von Fettalkoholpolyglycolethern in Form der Alkali- oder Alkanolammoniumsalze.

Neben den genannten Bestandteilen kann die diskontinuierliche Ölphase noch öllösliche Wirkstoffe wie z. B. Lichtschutzmittel, Antioxydantien, Vitamine, oder öllösliche Konservierungsmittel, z. B. p-Hydroxybenzoesäurebenzylester enthalten.

Die kontinuierliche wäßrige Phase kann neben der erfindungsgemäß enthaltenen Kombination von Hydrokolloiden noch weitere wasserlösliche Hilfsmittel enthalten. Als solche können z. B. Polyole wie Glycerin oder Sorbit, wasserlösliche Salze, z. B. Magnesiumsulfat, Puffersubstanzen, z. B. Alkaliphosphat, Alkalicitrat, Borate, wasserlösliche Konservierungsmittel wie z. B. p-Hydroxybenzoesäuremethylester, Sorbinsäure, wasserlösliche oberflächenaktive Stoffe oder Emulgatoren, wasserlösliche Farbstoffe oder wasserlösliche kosmetische Wirkstoffe, z. B. wasserlösliche Pflanzenextrakte, wasserlösliche Proteine oder Proteinderivate, Aminosäuren usw. genannt werden.

Die erfindungsgemäßen Kosmetische Zubereitungen enthalten O/W-Emulsionen mit einer Ölphase und einer wäßrigen Phase bevorzugt in einem Gewichtsverhältnis von 1 : 9 bis 4 : 6. Die erfindungsgemäß einzusetzende Kombination von Hydrokolloiden sollte in einer Menge von ca. 0,2 bis 2,0 Gew.-%, bezogen auf die gesamte Emulsion, enthalten sein. Das Mengenverhältnis von Celluloseether (a) zu Acrylsäure-Polymerisat (b) liegt bevorzugt bei (a) : (b) = 1 : 3 bis 3 : 1, insbesondere bei ca. 1 : 1. Besonders gute und kosmetisch befriedigende Zubereitungen werden erhalten, wenn die Kombination der Hydrokolloide in einer Menge von 0,3 - 1,0 Gew.-%, bezogen auf die gesamte Emulsion, enthalten ist.

Die erfindungsgemäßen Kosmetischen Zubereitungen können von flüssiger oder von cremiger Konsistenz sein. In jedem Falle zeigen die O/W-Emulsionen ein besseres Viskositätsverhalten und machen beim Auftragen und Verreiben auf der Haut einen kosmetisch besseren, gehaltvolleren Eindruck als entsprechende Emulsionen, die nur eine der beiden Hydrokolloid-Komponenten alleine enthalten. Zur weiteren Erläuterung der Erfindung werden folgende Beispiele gegeben:

**Beispiele**

1. Viskositätsverhalten wäßriger Lösungen von Hydrokolloiden Es wurde die Viskosität folgender wäßriger Lösungen bei verschiedenen Schergeschwindigkeiten gemessen

(a) 0,5 Gew.-% Viscontran [(R)] MHPC 3000
99,5 Gew.-% Wasser
(b) 0,5 Gew.-% Carbopol [(R)] 940
99,5 Gew.-% Wasser, Triethanolamin bis pH = 7
(c) 0,5 Gew.-% Viscontran [(R)] MHPC 3000
0,5 Gew.-% Carbopol [(R)] 940
99,0 Gew.-% Wasser, Triethanolamin bis pH = 7

Die Viskositätsmessungen wurden mit einem Haake-Rotovisko Rotationsviskosimeter bei 20 °C durchgeführt. Aus den bei den verschiedenen Schergeschwindigkeiten gemessenen Schubspannungen wurden folgende Viskositäten errechnet:

| Schergeschwindigkeit $(s^{-1})$ | Viskosität der Lösung (mPas) | | |
|---|---|---|---|
| | a | b | c |
| 10 | 140 | 18 000 | 25 400 |
| 28 | 135 | 9 200 | 14 000 |
| 56 | 130 | 5 400 | 8 500 |
| 112 | 120 | 3 100 | 5 000 |

Man erkennt, daß die Lösung c eine synergistisch gesteigerte Viskosität im gesamten Scherbereich aufweist, die bei niedrigen Schergeschwindigkeiten besonders stark hervortritt.

2. Anwendungsbeispiele

### 2.1 Nachtcreme, O/W

| | | |
|---|---|---|
| Cutina [(R)] MD [1)] | 8,0 | Gew.-% |
| Cetylalkohol | 2,0 | " |
| Eumulgin [(R)] B1 [2)] | 1,0 | " |
| Eumulgin [(R)] B2 [3)] | 1,0 | " |
| 2-Octyl-dodecanol | 8,0 | " |
| Myritol [(R)] 318 [5)] | 3,0 | " |
| Paraffinöl, dünnflüssig | 3,0 | " |
| Hydagen [(R)] F [6)] | 0,2 | " |
| Viscontran [(R)] MHPC 6000 [7)] | 0,2 | " |
| Carbopol [(R)] 940 [10)] | 0,4 | " |
| Triethanolamin | 0,8 | " |
| Wasser | ad 100,0 | " |

### 2.2 Feuchtigkeitscreme, O/W

| | | |
|---|---|---|
| Cetyl-stearylalkohol (50 : 50) | 8,0 | Gew.-% |
| Palmitin-Stearinsäure (50 : 50) | 4,0 | " |
| Eumulgin [(R)] B3 [4)] | 1,5 | " |
| Paraffinöl, dickflüssig | 4,0 | " |
| 2-Octyl-dodecanol | 8,0 | " |
| Hydagen [(R)] F [6)] | 1,0 | " |
| Triethanolamin | 1,8 | " |
| Carbopol [(R)] 940 [10)] | 0,5 | " |
| Viscontran [(R)] MHPC 3000 [8)] | 0,5 | " |
| Wasser | ad 100,0 | " |

2.3 Pflegecreme, O/W

| | |
|---|---|
| Cutina (R) KD16 13) | 5,0 Gew.-% |
| 2-Octyl-dodecanol | 4,0 " |
| Paraffinöl, dünnflüssig | 4,0 " |
| Palmitin-Stearinsäure (50 : 50) | 3,0 " |
| Cetylalkohol | 2,0 " |
| Glycerin, 86gew.-%ig | 3,0 " |
| Kaliumhydroxid | 0,25 " |
| Carbopol (R) 934 11) | 0,2 " |
| Viscontran (R) MHPC 6000 7) | 0,3 " |
| Wasser | ad 100,0 " |

2.4 Körperlotion, O/W

| | |
|---|---|
| Cutina (R) MD 1) | 4,0 Gew.-% |
| Palmitin-Stearinsäure (50 : 50) | 4,0 " |
| Cetyl-stearylalkohol (50 : 50) | 2,0 " |
| Eumulgin (R) B2 3) | 2,0 " |
| 2-Octyl-dodecanol | 3,0 " |
| Myritol (R) 318 5) | 3,0 " |
| Carbopol (R) 940 10) | 0,2 " |
| Viscontran (R) MHPC 3000 8) | 0,6 " |
| Triethanolamin | 1,5 " |
| Wasser | ad 100,0 " |

2.5 Reinigungsemulsion, O/W

| | |
|---|---|
| Cutina (R) MD 1) | 5,0 Gew.-% |
| Palmitin-Stearinsäure (50 : 50) | 5,0 " |
| Cetyl-stearylalkohol (50 : 50) | 2,0 " |
| Eumulgin (R) B1 2) | 1,0 " |
| Eumulgin (R) B2 3) | 1,0 " |
| 2-Octyl-dodecanol | 10,0 " |
| Myritol (R) 318 5) | 5,0 " |
| Carbopol (R) 940 10) | 0,4 " |
| Viscontran (R) MHPC 6000 7) | 0,2 " |
| Triethanolamin | 1,5 " |
| Wasser | ad 100,0 " |

## 2.6 Feuchtigkeitsemulsion, O/W

| | |
|---|---|
| Palmitin-Stearinsäure (50 : 50) | 4,0 Gew.-% |
| Cetyl-Stearylalkohol (50 : 50) | 2,0 " |
| Eumulgin (R) B2 [3] | 2,0 " |
| Isopropylmyristat | 5,0 " |
| 2-Octyldodecanol | 2,0 " |
| Myritol (R) 318 [5] | 3,0 " |
| Hydagen (R) F [6] | 3,0 " |
| Carbopol (R) 941 [12] | 0,7 " |
| Viscontran (R) MHPC 3000 [8] | 0,6 " |
| Triethanolamin | 1,5 " |
| Wasser | ad 100,0 " |

## 2.7 Pflegemilch, O/W

| | |
|---|---|
| Paraffinöl, dünnflüssig | 4,0 Gew.-% |
| Hostaphat (R) KW 340 N [9] | 4,0 " |
| 2-Octyl-dodecanol | 8,0 " |
| Cetyl-stearylalkohol | 1,0 " |
| Eumulgin (R) B1 [2] | 1,0 " |
| Glycerin, 99,5%ig | 1,0 " |
| Triethanolamin | 0,15 " |
| Pflanzenextrakte, wäßrig | 10,0 " |
| Carbopol (R) 940 [10] | 0,2 " |
| Viscontran (R) MHPC 3000 [8] | 0,3 " |
| Wasser | ad 100,0 " |

In den Rezepturen 2.1 bis 2.7 wurden folgende Warenzeichen verwendet:

| | |
|---|---|
| 1) Cutina (R) MD: | Gemisch von Mono- und Diglyceriden der Palmitin- und Stearinsäure (Henkel KGaA) |
| 2) Eumulgin (R) B1: | Cetyl-stearylalkohol + 12 Mol Ethylenoxid (Henkel KGaA) |
| 3) Eumulgin (R) B2: | Cetyl-stearylalkohol + 20 Mol Ethylenoxid (Henkel KGaA) |
| 4) Eumulgin (R) B3: | Cetyl-stearylalkohol + 30 Mol Ethylenoxid (Henkel KGaA) |
| 5) Myritol (R) 318: | Capryl-caprinsäure-triglycerid (Henkel KGaA) |
| 6) Hydagen (R) F: | Natriumsalz einer Polyhydroxycarbonsäure (Henkel KGaA) |
| 7) Viscontran (R) MHPC 6000: | Methylhydroxypropylcellulose, Viskosität 2%ig in destilliertem Wasser (20 °C, Brookfield-Viskosimeter, 20 UpM) 5 200 -6 500 mPas (Henkel KGaA) |
| 8) Viscontran (R) MHPC 3000: | Methylhydroxypropylcellulose, Viskosität 2%ig in destilliertem Wasser (20 °C, Brookfield-Viskosimeter, 20 UpM) 2 900 -4 400 mPas (Henkel KGaA) |

9) Hostaphat KW 340 N:  Phosphorsäureester eines Wachsalkohol + 4 Mol Ethylenoxid-Addukts (Hoechst AG)

10) Carbopol (R) 940:  Vernetztes Acrylsäure-Polymerisat, mittleres Molekulargewicht ca. 4 000 000 (B. F. Goodrich Chem. Comp. Avon Lake, Ohio, USA)

11) Carbopol (R) 934:  Vernetztes Acrylsäure-Polymerisat, mittleres Molekulargewicht ca. 3 000 000 (B. F. Goodrich Chem. Comp.)

12) Carbopol (R) 941:  Vernetztes Acrylsäure-Polymerisat, mittleres Molekulargewicht ca. I 250 000 (B. F. Goodrich Chem. Comp.)

13) Cutina (R) KD 16:  Gemisch aus Mono- und Diglyceriden höherer gesättigter Fettsäuren mit Kaliumstearat

## Patentansprüche

**1.** Kosmetische Zubereitung für die topische Anwendung in Form einer Öl-in-Wasser-Emulsion, die einen wasserlöslichen nichtionischen Celluloseether und ein vernetztes Acrylsäure-Polymerisat oder -Copolymerisat enthält, dadurch gekennzeichnet, daß die kontinuierliche wäßrige Phase eine Kombination von Verdickungsmitteln aus

(a) Methylhydroxypropylcellulose mit einer Brookfield-Viskosität von 40 bis 40 000 mPas, gemessen bei 20 UpM, einer 2 Gew.-%igen wäßrigen Lösung des Celluloseethers bei 20 °C und

(b) einem wasserlöslichen Salz eines vernetzten AcrylsäurePolymerisats oder Acrylsäure-Copolymerisats mit einem mittleren Molekulargewicht von 1 000 000 bis 5 000 000 im Mengenverhältnis (a) : (b) = 9 : 1 bis 1 : 9

enthält.

## Claims

**1.** A cosmetic preparation for topical application in the form of an oil-in-water emulsion containing a water-soluble non-ionic cellulose ether and a cross-linked acrylic acid polymer or copolymer, characterized in that the continuous aqueous phase contains a combination of thickeners comprising

(a) methylhydroxypropyl cellulose having a Brookfield viscosity of from 40 to 40,000 mPa·s, measured at 20 r.p.m. in a 2% by weight aqueous solution of the cellulose ether at 20 °C, and

(b) a water-soluble salt of a cross-linked acrylic acid polymer or acrylic acid copolymer having an average molecular weight of from 1,000,000 to 5,000,000

in a quantitative ratio of (a):(b) = 9:1 to 1:9.

## Revendications

**1.** Composition cosmétique pour l'utilisation topique sous forme d'une émulsion huile dans l'eau, qui renferme un éther de cellulose non ionique soluble dans l'eau et un polymérisat ou copolymérisat d'acide acrylique, caractérisé en ce que la phase aqueuse continue contient une combinaison d'agent d'épaississement à base

(a) de méthylhydroxypropylcellulose ayant une viscosité Brookfield de 40 à 40.000 mPa.s mesurée à 20 t/mn d'une solution aqueuse à 2 % en poids d'éther de cellulose à 20 °C et

(b) d'un sel soluble dans l'eau d'un polymérisat ou copolymérisat d'acide acrylique réticulé ayant un poids moléculaire moyen allant de 1.000.000 à 5.000.000 avec un rapport pondéral (a) : (b) = 9 : 1 à 1 : 9.